Europäisches Patentamt

European Patent Office

Office européen des brevets

(19)

(11) **EP 1 466 887 A1**

(12) **EUROPÄISCHE PATENTANMELDUNG**

(43) Veröffentlichungstag:
**13.10.2004 Patentblatt 2004/42**

(51) Int Cl.⁷: **C07C 209/28**, C07C 211/35,
C07C 211/08, C07C 211/21,
C07C 211/27

(21) Anmeldenummer: **04007896.6**

(22) Anmeldetag: **01.04.2004**

(84) Benannte Vertragsstaaten:
**AT BE BG CH CY CZ DE DK EE ES FI FR GB GR
HU IE IT LI LU MC NL PL PT RO SE SI SK TR**
Benannte Erstreckungsstaaten:
**AL HR LT LV MK**

(30) Priorität: **10.04.2003 DE 10316375**

(71) Anmelder: **Celanese Chemicals Europe GmbH
61476 Kronberg (DE)**

(72) Erfinder:
• **Lappe, Peter, Dr.
46539 Dinslaken (DE)**
• **Springer, Helmut
46539 Dinslaken (DE)**

(54) **Verfahren zur Herstellung von N-Methyl-Dialkylaminen aus sekundären Dialkylaminen und Formaldehyd**

(57) Die vorliegende Erfindung betrifft ein Verfahren zur Herstellung von N-Methyl-dialkylaminen durch Umsetzung von sekundären Dialkylaminen mit Formaldehyd bei einer Temperatur von 100 bis 200°C, wobei man je Mol sekundärem Dialkylamin 1,5 bis 3 Mol Formaldehyd einsetzt und das erhaltene Reaktionsprodukt entgast, die wässrige Phase abtrennt und die organische Phase destilliert.

EP 1 466 887 A1

**Beschreibung**

Die vorliegende Erfindung betrifft ein Verfahren zur Herstellung von N-Methyl-dialkylaminen aus sekundären Aminen und Formaldehyd.

Tertiäre Amine und insbesondere N-Methyl-dialkylamine besitzen als wichtige Zwischenprodukte für die chemische Industrie eine große wirtschaftliche Bedeutung. Sie werden als Vulkanisationsbeschleuniger in der Gummiindustrie oder als Polymerisations- und Härtungskatalysatoren für die Herstellung von Kunststoffformkörpern auf Epoxid- und Polyurethanbasis eingesetzt. Ferner sind sie als Korrosionsinhibitoren und als Ausgangssstoffe für Detergentien und Flockungsmittel geeignet. N-Methyl-dialkylamine haben ebenfalls Bedeutung als wertvolle Zwischenprodukte für die Herstellung von pharmazeutischen Produkten oder von Substanzen erlangt, die im Pflanzenschutzbereich eingesetzt werden.

Aufgrund der hohen technischen Bedeutung von N-Methyl-dialkylaminen finden sich in der wissenschaftlichen und technischen Literatur zahlreiche Hinweise für ihre Herstellung.

Es ist bekannt, tertiäre Amine ausgehend von sekundären Aminen durch Umsetzung mit Carbonylverbindungen in Gegenwart von Ameisensäure nach Leuckart-Wallach herzustellen (Houben-Weyl; Seite 648; Methodicum Chimicum S.541). Ameisensäure dient als Reduktionsmittel und wird meist in Form von Formamid oder Ammoniumformiat eingesetzt.

Geht man von Formaldehyd als Carbonylkomponente aus, so führt die in Gegenwart von Ameisensäure durchgeführte Reaktion mit sekundären Aminen zu N-Methyl-dialkylaminen. Bei dieser nach Eschweiler-Clarke benannten Methode setzt man pro Mol Amin gewöhnlich 1-1,25 Mol Formaldehyd und 2-4 Mol Ameisensäure ein (Methodicum Chimicum, Band 6, S. 543, Georg Thieme Verlag, Stuttgart 1974). Eine neuere Weiterentwicklung dieses Verfahrens besteht in der N-Methylierung von Aminen unter MikrowellenBestrahlung (Synthetic Communications (1996), 26 (21), 3919-3922).

Aliphatische sekundäre Amine können auch durch Erhitzen mit Formaldehyd auf 120-160°C zu tertiären Aminen methyliert werden. Nach Hoppe Seyler's Zeitschrift für Physiologische Chemie, Walter de Gruyter & Co., 196, 1931, Seiten 81-86, läßt sich die Methylierungsreaktion in Gegenwart 10%iger Formaldehydlösung unter Calciumchloridzusatz oder unter Verwendung von Paraformaldehyd durchführen. Nach dem bekannten Verfahren wird das aliphatische sekundäre Amin in Form seines Hydrochlorids eingesetzt. Auch wird ein hoher Formaldehydzusatz benötigt, wobei je Mol aliphatischem sekundären Amin bis zu 42 Mol Paraformaldehyd eingesetzt werden.

Auch nach Berichte 45, 1912, Seiten 2404-2409 erhält man durch Einwirken einer 40%igen Lösung von Formaldehyd auf Phenylethylamin-chlorhydrat N-Dimethylphenylethylamin.

In der Literatur werden zudem noch weitere zahlreiche Methoden zur Herstellung von N-Methyl-dialkylaminen beschrieben. So kann zum Beispiel N-Methyl-di-n-propylamin durch Umsetzung von Di-n-propylamin mit Methyliodid in guten Ausbeuten erhalten werden (Chem. Ber. 33, 1900, 1439-1440). Anstelle von Methyliodid ist auch die Verwendung von Dimethylsulfat als Alkylierungsmittel (J. Chem. Soc. 105, 1914, 2766) oder von Methyltrimethoxyphosphoniumtetrafluoroborat (J. Org. Chem. 49 (25), 1984, 4877-4880) beschrieben.

Im industriellen Maßstab erfolgt die Herstellung von N-Methyl-dialkylaminen aus Formaldehyd und sekundären Aminen durch katalytische Hydrierung mit Wasserstoff am Metallkontakt, beispielsweise an Nikkel- oder Palladiumkatalysatoren.

Nach US 4,757,144 erfolgt die Herstellung von tertiären Aminen aus primären oder sekundären Aminen und Formaldehyd durch eine Suspensionshydrierung in Gegenwart von Palladium oder Platinkontakten. Die Hydrierung wird bei einer Temperatur von 80°C bis 180°C und bei einem Druck von 0,29 bis 4,9 MPa Überdruck durchgeführt.

EP-A1-0 492 771 beschreibt ebenfalls ein katalytisches Verfahren zur Herstellung von N-Methyl-dialkylaminen ausgehend von sekundären Alkylaminen in Gegenwart von Formaldehyd. Die Reaktionspartner werden bei einem Druck von 0,8 bis 28 MPa und einer Temperatur von 100 bis 150°C am Festbettkontakt zur Reaktion gebracht. Als Katalysator wird ein mit Übergangsmetallen, vorzugsweise ein mit Kupfer und Chrom dotierter Nickelkontakt eingesetzt.

Auch DE-A1-35 44 510 befasst sich mit der Herstellung aliphatischer tertiärer Amine ausgehend von primären oder sekundären Aminen durch Reaktion mit Formaldehyd. Die zunächst gebildeten N-Methylolamine werden anschließend bei Temperaturen von 50 bis 200°C und Drücken von 0,1 bis 10 MPa in Suspension an feinverteilten Palladium- oder Palladiumkatalysatoren hydriert.

Die bekannten Verfahren zur Herstellung von N-Methyl-dialkylaminen ausgehend von sekundären Alkylaminen und Formaldelyd erfordern entweder die Anwesenheit eines speziellen zugesetzten Reduktionsmittels, beispielsweise von Ameisensäure, oder eines Hydrierkatalysators und Wasserstoff. Beide Verfahrensvarianten sind aufwendig, denn die Verwendung spezieller Reduktionsmittel, wie Ameisensäure, erfordert aufgrund ihrer korrosiven Wirkung speziell ausgelegte Apparatewerkstoffe. Auch die Bereitstellung einer mit Wasserstoff durchgeführten katalytischen Hydrierstufe ist aufwendig und erfordert zusätzlich eine Wasserstoffquelle sowie eine Versorgung mit metallhaltigen Hydrierkatalysatoren. Ebenfalls ist für die Entsorgung der erschöpften Hydrierkatalysatoren Sorge zu tragen. Daher besteht ein Bedarf nach einem möglichst einfachen und kostengünstigen Verfahren zur Herstellung von N-Methyl-dialkylaminen.

Überraschenderweise wurde gefunden, dass sekundäre Alkylamine bei nur geringem Überschuß an Formaldehyd in sehr hohen Ausbeuten zu den entsprechenden N-Methyl-dialkylaminen umgesetzt werden können.

Die Erfindung besteht daher in einen Verfahren zur Herstellung von N-Methyl-di-alkylaminen aus sekundären Dialkylaminen und Formaldehyd bei einer Temperatur von 100 bis 200°C. Es ist dadurch gekennzeichnet, dass man pro Mol sekundärem Dialkylamin 1,5 bis 3 Mol Formaldehyd einsetzt, das erhaltene Reaktionsprodukt entgast, die wässrige Phase abtrennt und die organische Phase destilliert.

Überraschenderweise lassen sich bei dem gewählten Molverhältnis Dialkylamin zu Formaldehyd die gewünschten N-Methyl-dialkylamine auf einfache Weise in hohen Ausbeuten erhalten. Ohne das Reaktionsgeschehen mechanistisch deuten zu wollen wird angenommen, dass die Umsetzung von einem Mol sekundärem Alkylamin mit 1,5 Mol Formaldehyd nach folgender Stöichiometrie abläuft:

$$2\ R_2NH + 3\ HCHO \rightarrow 2R_2N\text{-}CH_3 + CO_2 + H_2O$$

Für die Durchführung des beanspruchten Verfahrens ist es daher wesentlich, die Menge an Formaldehyd, die über der stöchiometrisch erforderlichen Menge liegt, auf einen engen Bereich zu begrenzen, um die Bildung unerwünschter Nebenprodukte zu unterdrücken und den Verlust an Ausgangsstoffen gering zu halten. Pro Mol an sekundärem Alkylamin sind 1,5 bis 3, vorzugsweise 1,5 bis 2,5 Mol Formaldehyd einzusetzen.

Das beanspruchte Verfahren wird bei einer Temperatur von 100 bis 200°C, vorzugsweise von 120 bis 160°C durchgeführt. Man arbeitet bei Eigendruck, der sich infolge der gewählten Reaktionstemperatur einstellt. Die Reaktion kann kontinuierlich oder diskontinuierlich gefahren werden.

Nach beendeter Reaktion wird das Wasser und die organische Phase enthaltende Reaktionsprodukt zunächst abgeführt und dann entgast, um gebildetes $CO_2$ auszutreiben. Dazu wird das Rohgemisch abgekühlt und gegebenenfalls nach Zusatz eines organischen Lösungsmittels, wie Isopropanol, unter Normaldruck bei erhöhter Temperatur behandelt, gegebenenfalls auch destilliert. Der zu wählende Temperaturbereich hängt von der Siedelage der organischen Produkte, insbesondere von den Siedepunkten des Dialkylamins und des gewünschten N-Methyl-dialkylamins ab und liegt im allgemeinen in einem Temperaturbereich von 50 bis 150°C.

Nach der Entgasung des Reaktionsgemisches wird die organische Produktphase von der wässrigen Phase geschieden, beispielsweise durch Überleiten über eine Membran oder durch einfache Phasentrennung, gegebenenfalls nach Zusatz eines organischen Lösungsmittels wie n-Hexen-1 zur Unterstützung der Phasentrennung. Danach folgt die fraktionierte Destillation des vom Wasser befreiten Rohproduktes unter konventionellen Bedingungen.

Formaldehyd kommt üblicherweise als wässrige Lösung mit einer Konzentration von 10 bis 60 Gew.-% zum Einsatz. Vorzugsweise verwendet man eine wässrige Formaldehydlösung mit einem Gehalt von 25 bis 40 Gew.-% Formaldehyd.

Das sekundäre Dialkylamin wird in unverdünnter Form ohne Lösungsmittelzusatz zugegeben. Als sekundäre Dialkylamine kommen gemischte oder symmetrische cycloaliphatische oder aliphatische Dialkylamine mit geradkettigen oder verzweigten, gesättigten oder ungesättigten Alkylgruppen mit jeweils 2 bis 20 Kohlenstoffatomen, insbesondere 2 bis 15, bevorzugt 2 bis 9 Kohlenstoffatomen, oder mit Arylalkylgruppen mit jeweils 7 bis 15 Kohlenstoffatomen in Betracht.

Stellvertretend für cycloaliphatische sekundäre Amine sind Dicyclohexylamin und für aliphatische sekundäre Amine Di-n-propylamin, Di-n-butylamin, Di-n-pentylamin, Di-2-ethylhexylamin und Diisononylamin zu nennen.

Aber auch gemischte cycloaliphatische, aliphatische Dialkylamine wie N-Ethylcyclohexylamin, lassen sich nach dem erfindungsgemäßen Verfahren in die tertiären Amine überführen. Stellvertretend für sekundäre Dialkylamine mit ungesättigten Alkylgruppen steht Diallylamin. Dibenzylamin steht beispielhaft für ein sekundäres Dialkylamin mit Arylalkylgruppen.

Das erfindungsgemäße Verfahren ist besonders zur Herstellung von N-Methyl-di-n-butylamin und N-Methyl-di-n-propylamin geeignet.

Die beanspruchte Arbeitsweise gestattet einen eleganten und einfachen Zugang zu N-Methyl-dialkylaminen in überraschend hohen Ausbeuten und als Nebenprodukte fallen nur Wasser und Kohlendioxid an, die problemlos aus dem Reaktionsgemisch entfernt werden können. Hingegen gestalten sich die bekannten Verfahren durchweg komplexer und liefern die gewünschten N-Methyl-dialkylamine in meist geringeren Ausbeuten.

Die nachfolgend aufgeführten Beispiele belegen die Erfindung, ohne sie einzuschränken.

Beispiel 1

Herstellung von N-Methyl-diethylamin

149,3 g (2,0 mol) Diethylamin (98 %-ig) und 330,2 g (3,2 mol) Formaldehyd (29,1 %-ig) werden in einem 1l-Autoklav vorgelegt und auf 120°C erwärmt. Die Reaktionszeit bei dieser Temperatur beträgt 8 Std., wobei sich ein Druck von max. 1,44 MPa einstellt. Danach wird das Reaktionsgemisch abgekühlt und destillativ aufgearbeitet.

Dazu wird das Rohamin (wässrige und organische Phase) mit 200,0 g Isopropanol versetzt, in einen 1 l Kolben überführt und unter Rühren langsam auf 60°C erwärmt. Dabei beginnt eine leichte Gasentwicklung, die beim Erreichen einer Temperatur von ca. 75°C beendet ist. Danach wird unter Normaldruck vom Rückstand abdestilliert, es wird eine Destillatmenge von 614,4 g isoliert. Dieses Destillat wird zur Wasserabtrennung über eine Membrane geleitet und anschließend fraktioniert destilliert, das Amin siedet bei 63 - 65°C. Es werden 168,8 g Wertprodukt mit einer Reinheit von 96,0 % isoliert, dies entspricht einer Ausbeute von 93,0 % d. Th.

Beispiel 2

Herstellung von N-Methyl-di-n-butylamin

Analog Beispiel 1 werden 258,5 g (2,0 mol) Di-n-butylamin und 330,2 g (3,2 mol) Formaldehyd (29,1 %-ig) in einem 1l-Autoklav umgesetzt, dabei stellt sich bei einer Temperatur von 120°C ein Druck von max. 3,2 MPa ein. Nach Entgasung des Rohamins (wässrige und organische Phase) bei einer Temperatur von max. 80°C, erfolgt die Abtrennung des Wassers an einem Wasserabscheider. Die verbleibende organische Phase wird anschließend fraktioniert destilliert. Es werden 274,6 g N-Methyl-di-n-butylamin mit einer Reinheit von 99,6 % isoliert, dies entspricht einer Ausbeute von 95,6 % d.Th.

Beispiel 3

Herstellung von N-Methyl-N-ethyl-n-butylamin

Analog Beispiel 1 werden 202,4 g (2,0 mol) N-Ethyl-n-butylamin und 330,2 g (3,2 mol) Formaldehyd (29,1 %-ig) in einem 1l-Autoklav umgesetzt, dabei stellt sich bei einer Temperatur von 120°C ein Druck von max. 2,2 MPa ein. Nach Entgasung des Rohamins (wässrige und organische Phase) bei einer Temperatur von max. 88°C, erfolgt die Abtrennung des Wassers an einem Wasserabscheider. Die verbleibende organische Phase wird anschließend fraktioniert destilliert. Es werden 223,0 g N-Methyl-N-ethyl-n-butylamin mit einer Reinheit von 97,8 % isoliert, dies entspricht einer Ausbeute von 94,7 % d.Th.

Beispiel 4

Herstellung von N-Methyl-N-Ethyl-1.2-dimethylpropylamin

Analog Beispiel 1 werden 230,4 g (2,0 mol) N-Ethyl-1.2-dimethylpropylamin und 330,2 g (3,2 mol) Formaldehyd (29,1%-ig) in einem 1l-Autoklav umgesetzt, dabei stellt sich bei einer Temperatur von 120°C ein Druck von max. 2,3 MPa ein. Nach Entgasung des Rohamins (wässrige und organische Phase) bei einer Temperatur von max. 94°C, erfolgt die Abtrennung des Wassers an einem Wasserabscheider. Die verbleibende organische Phase wird anschließend fraktioniert destilliert. Es werden 251,7 g N-Methyl-N-ethyl-1.2-dimethylpropylamin mit einer Reinheit von 98,6 % isoliert, dies entspricht einer Ausbeute von 96,0 % d.Th.

Beispiel 5

Herstellung von N-Methyl-diamylamin

Analog Beispiel 1 werden 314,4 g (2,0 mol) Diamylamin (technisches Gemisch aus 18,3 Gew.-% Diisopentylamin, 61,0 Gew.-% Di-n/i-pentylamin und 19,2 Gew.-% Di-n-pentylamin, Rest: 1,5 Gew.-%) und 330,2 g (3,2 mol) Formaldehyd (29,1 %-ig) in einem 1l-Autoklav umgesetzt, dabei stellt sich bei einer Temperatur von 120°C ein Druck von max. 2,5 MPa ein. Nach Entgasung des Rohamins (wässrige und organische Phase) bei einer Temperatur von max. 102°C erfolgt die Abtrennung des Wassers an einem Wasserabscheider. Die verbleibende organische Phase wird anschließend destilliert. Es werden 329,1 g N-Methyl-diamylamin mit einer Reinheit von 91,8 % isoliert, dies entspricht einer Ausbeute von 88,3 % d.Th.

Beispiel 6

Herstellung von N-Methyl-di-2-ethylhexylamin

Analog Beispiel 1 werden 181,1 g (0,75 mol) Di-2-ethylhexylamin und 193,5 g (1,88 mol) Formaldehyd (29,1 %-ig) in einem 1l-Autoklav umgesetzt, dabei stellt sich bei einer Temperatur von 160°C ein Druck von max. 1,9 MPa ein. Nach Entgasung des Rohamins (wässrige und organische Phase) bei einer Temperatur von max. 100°C, erfolgt die Abtrennung des Wassers unter Zusatz von 50 g n-Hexen-1 an einem Wasserabscheider. Die verbleibende organische Phase wird anschließend destilliert. Es werden 185,1 g N-Methyldi-2-ethylhexylamin mit einer Reinheit von 91,4 % isoliert, dies entspricht einer Ausbeute von 88,3 % d.Th.

Beispiel 7

Herstellung von N-Methyl-diisononylamin

Analog Beispiel 1 werden 202,1 g (0,75 mol) Di-isononylamin und 170,3 g (1,65 mol) Formaldehyd (29,1 %-ig) in einem 1l-Autoklav umgesetzt, dabei stellt sich bei einer Temperatur von 160°C ein Druck von max. 1,9 MPa ein. Nach Reaktionsende wird die organische Phase von der Wasserphase abgetrennt, die verbleibende organische Phase wird anschließend destilliert. Es werden 208,0 g N-Methyl-diisononylamin mit einer Reinheit von 85,0 % isoliert, dies entspricht einer Ausbeute von 83,2 % d.Th.

Beispiel 8

Herstellung von N-Methyl-N-ethylcyclohexylamin

Analog Beispiel 1 werden 190,8 g (1,5 mol) N-Ethylcyclohexylamin und 247,6 g (2,4 mol) Formaldehyd (29,1 %-ig) in einem 1l-Autoklav umgesetzt, dabei stellt sich bei einer Temperatur von 160°C ein Druck von max. 2,6 MPa ein. Nach Reaktionsende werden 30 g Hexen-1 zugesetzt und die organische Phase wird von der Wasserphase abgetrennt. Die verbleibende organische Phase wird anschließend fraktioniert destilliert. Es werden 196,9 g N-Methyl-N-ethylcylcohexylamin mit einer Reinheit von 98,7 % isoliert, dies entspricht einer Ausbeute von 91,8% d.Th.

Beispiel 9

Herstellung von N-Methyl-dibenzylamin

Analog Beispiel 1 werden 197,3 g (1,0 mol) Dibenzylamin und 165,1 g (1,6 mol) Formaldehyd (29,1 %-ig) in einem 1l-Autoklav umgesetzt, dabei stellt sich bei einer Temperatur von 160°C ein Druck von max. 1,8 MPa ein. Nach Reaktionsende werden 30 g Hexen-1 zugesetzt und die organische Phase wird von der Wasserphase abgetrennt. Die verbleibende organische Phase wird anschließend destilliert. Es werden 191,8 g N-Methyl-dibenzylamin mit einer Reinheit von 93,8 % isoliert, dies entspricht einer Ausbeute von 85,1 % d.Th.

Beispiel 10

Herstellung von N-Methyl-dicyclohexylamin

Analog Beispiel 1 werden 204,0 g (1,13 mol) Dicyclohexylamin und 185,5 g (1,80 mol) Formaldehyd (29,1 %-ig) in einem 1l-Autoklav umgesetzt, dabei stellt sich bei einer Temperatur von 160°C ein Druck von max. 2,4 MPa ein. Nach Reaktionsende werden 50 g Hexen-1 zugesetzt und die organische Phase wird von der Wasserphase abgetrennt. Die verbleibende organische Phase wird anschließend destilliert. Es werden 226,3 g N-Methyl-dicyclohexylamin mit einer Reinheit von 93,7 % isoliert, dies entspricht einer Ausbeute von 96,1 % d. Th.

Beispiel 11

Herstellung von N-Methyl-diallylamin

Analog Beispiel 1 werden 194,3 g (2,0 mol) Diallylamin und 330,2 g (3,2 mol) Formaldehyd (29,1 %-ig) in einem 1l-Autoklav umgesetzt, dabei stellt sich bei einer Temperatur von 120°C ein Druck von max. 2,5 MPa ein. Nach Entgasung des Rohamins (wässrige und organische Phase) bei einer Temperatur von max. 87°C, erfolgt die Abtrennung des Wassers an einem Wasserabscheider. Die verbleibende organische Phase wird anschließend fraktioniert destilliert. Es werden 216,3 g N-Methyl-diallylamin mit einer Reinheit von 95,7 % isoliert, dies entspricht einer Ausbeute von 93,1 % d. Th.

**Patentansprüche**

1. Verfahren zur Herstellung von N-Methyl-dialkylaminen aus sekundären Dialkylaminen und Formaldehyd bei einer Temperatur von 100 bis 200°C, **dadurch gekennzeichnet, dass** man pro Mol sekundärem Dialkylamin 1,5 bis 3 Mol Formaldehyd einsetzt, das erhaltene Reaktionsprodukt entgast, die wässrige Phase abtrennt und die organische Phase destilliert.

2. Verfahren nach Anspruch 1, **dadurch gekennzeichnet, dass** man pro Mol sekundärem Dialkylamin 1,5 bis 2,5 Mol Formaldehyd einsetzt.

3. Verfahren nach Anspruch 1 oder 2, **dadurch gekennzeichnet, dass** man bei einer Temperatur von 120 bis 160°C arbeitet.

4. Verfahren nach einem oder mehreren der Ansprüche 1 bis 3, **dadurch gekennzeichnet, dass** man als sekundäre Dialkylamine gemischte oder symmetrische cycloaliphatische oder aliphatische Dialkylamine mit geradkettigen oder verzweigten, gesättigten oder ungesättigten Alkylgruppen mit jeweils 2 bis 20 Kohlenstoffatomen oder mit Arylalkylgruppen mit jeweils 7 bis 15 Kohlenstoffatomen einsetzt.

5. Verfahren nach Anspruch 4, **dadurch gekennzeichnet, dass** man als sekundäre Dialkylamine gemischte oder symmetrische cycloaliphatische oder aliphatische Dialkylamine mit geradkettigen oder verzweigten, gesättigten oder ungesättigten Alkylgruppen mit jeweils 2 bis 15 Kohlenstoffatomen, vorzugsweise 2 bis 9 Kohlenstoffatomen einsetzt.

6. Verfahren nach einem oder mehreren der Ansprüche 1 bis 3 zur Herstellung von N-Methyl-di-n-butylamin oder N-Methyl-di-n-propylamin.

**Europäisches Patentamt**

**EUROPÄISCHER RECHERCHENBERICHT**

Nummer der Anmeldung

EP 04 00 7896

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | F. F. BLICKE, F. B. ZIENTY: "Antispasmodics II" JOURNAL OF THE AMERICAN CHEMICAL SOCIETY, Bd. 61, 1939, Seiten 93-95, XP002291315 Experimental part, synthesis of compound No. 12 ----- | 1-5 | C07C209/28 C07C211/35 C07C211/08 C07C211/21 C07C211/27 |
| X | DE 80 520 C (W. ESCHWEILER) 1895 * Beispiel * ----- | 1-5 | |
| X | W. ESCHWEILER: "Ersatz von an Stickstoff gebundenen Wasserstoffatomen durch die Methylgruppe mit Hülfe von Formaldehyd" BERICHTE, Bd. 38, 1905, Seiten 880-882, XP009034778 * das ganze Dokument * ----- | 1-5 | |
| X | DE 287 802 C (FARBENFABRIKEN VORM FRIEDR. BAYER & CO.) 1915 * Beispiel 3 * ----- | 1-5 | |
| X | J. PLÖCHL: "Ueber eine Reaction des Formaldehyds" BERICHTE, Bd. 21, 1888, Seiten 2117-2119, XP009034275 * das ganze Dokument * ----- | 1-5 | **RECHERCHIERTE SACHGEBIETE (Int.Cl.7)** C07C |

Der vorliegende Recherchenbericht wurde für alle Patentansprüche erstellt

| Recherchenort | Abschlußdatum der Recherche | Prüfer |
|---|---|---|
| München | 6. August 2004 | Pérez Carlon, R |

EPO FORM 1503 03.82 (P04C03)

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

**EP 1 466 887 A1**

## ANHANG ZUM EUROPÄISCHEN RECHERCHENBERICHT
## ÜBER DIE EUROPÄISCHE PATENTANMELDUNG NR.

EP 04 00 7896

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-08-2004

| Im Recherchenbericht angeführtes Patentdokument | Datum der Veröffentlichung | Mitglied(er) der Patentfamilie | Datum der Veröffentlichung |
|---|---|---|---|
| DE 80520 C | | KEINE | |
| DE 287802 C | | KEINE | |